(19) Europäisches Patentamt
European Patent Office
Office européen des brevets

(11) Publication number: **0 167 155**
**B1**

(12) # EUROPEAN PATENT SPECIFICATION

(45) Date of publication of patent specification: **08.03.89**

(51) Int. Cl.$^4$: **C 07 F 7/10**, C 07 F 7/18, C 07 D 205/08

(21) Application number: **85108210.7**

(22) Date of filing: **03.07.85**

(54) **beta-Lactam compound and preparing thereof.**

(30) Priority: **05.07.84 JP 139797/84**

(43) Date of publication of application:
**08.01.86 Bulletin 86/02**

(45) Publication of the grant of the patent:
**08.03.89 Bulletin 89/10**

(84) Designated Contracting States:
**BE CH DE FR GB IT LI NL**

(56) References cited:
**EP-A-0 070 204**
**EP-A-0 078 026**
**EP-A-0 106 652**
**DE-B-1 770 855**

(73) Proprietor: **KANEGAFUCHI KAGAKU KOGYO KABUSHIKI KAISHA**
**2-4 Nakanoshima 3-chome**
**Kita-ku Osaka-shi Osaka-fu (JP)**

(72) Inventor: **Ohashi, Takehisa**
**9-14, Obanoyama-cho 3-chome Shinohara**
**Nada-ku Kobe-shi Hyogo-ken (JP)**
Inventor: **Kan, Kazunori**
**9-30-616, Maikodai 2-chome Tarumi-ku**
**Kobe-shi Hyogo-ken (JP)**
Inventor: **Sada, Isao**
**1459-1, Fujie**
**Akashi-shi Hyogo-ken (JP)**
Inventor: **Miyama, Akimasa**
**3-6-24, Okihama-cho Takasago-cho**
**Takasago-shi Hyogo-ken (JP)**
Inventor: **Watanabe, Kiyoshi**
**15-41, Matsugaoka 5-chome**
**Akashi-shi Hyogo-ken (JP)**

(74) Representative: **Türk, Gille, Hrabal**
**Bruckner Strasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 167 155 B1

**Description**

The present invention relates to a β-lactam compound having a hydroxyethyl group at the 3-position, where the hydroxy group is protected, and a silylether group at the 4-position, and a process for preparing thereof.

Since the β-lactam compound of the present invention has a highly reactive silylether group, it is a very useful intermediate which can be converted into various derivatives. For instance, 3-(1-hydroxyethyl)-4-acetoxyazetidin-2-one or 3-(1-hydroxyethyl)-4-haloazetidin-2-one, which are both useful for preparing thienamycin known as a β-lactam antibiotic of the fourth generation, can be obtained by the substitution reaction of the silylether group at the 4-position of the compound of the present invention.

The process for producing 4-acetoxy-3-(1-hydroxyethyl)-2-azetidinone is subject-matter of the claims of the co-pending EP 85 108 208.1 of the same effective date.

EP—A—70 204, EP—A—78 926 and EP—A—106 652 describe methods for the preparation of 4-acetoxy substituted 2-azetidinones starting from azetidinone-4-carboxylic acid derivatives and substituting an acetoxy group for the 4-carboxyl group by means of lead tetraacetate.

Hitherto there has not been known the β-lactam compound with a silylether group at the 4-position. Also, there has not been known a process for preparing the β-lactam compound having an O-protected hydroxyethyl group at the 3-position and a silylether group at the 4-position.

As the result of the continuous efforts of the inventors, it was found that the above β-lactam compound could be a useful intermediate for preparing carbapenem β-lactam compounds and the desired β-lactam ring could be formed by the reaction of an enolsilylether and chlorosulfonylisocyanate. And thus, the present invention has been completed.

An object of the present invention is to provide an easy process for preparing a β-lactam compound having a silylether group at the 4-position. According to the present invention, there can be provided a β-lactam compound having the general formula (I):

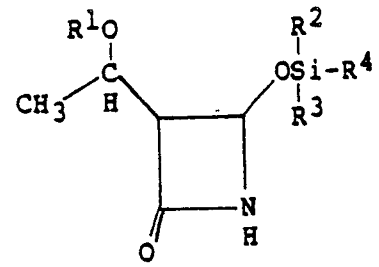

(I)

wherein $R^1$ is a protective group for hydroxy group, $R^2$ $R^3$ and $R^4$ are selected from an alkyl group of $C_1$ to $C_4$, a phenyl group and an aralkyl group, and a process for preparing thereof by reacting enolsilylethers having the general formula (III):

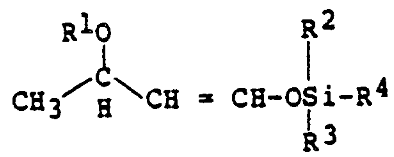

(III)

wherein $R^1$, $R^2$, $R^3$ and $R^4$ are as above, with chlorosulfonylisocyanate, followed by reduction.

In the above-mentioned general formula (I), $R^1$ is a protective group for hydroxy group such as the tert-butyldimethylsilyl group, triisopropylsilyl group, isopropyldimethylsilyl group, acetyl group, benzyloxycarbonyl group, o-nitrobenzyloxycarbonyl group, p-nitrobenzyloxycarbonyl group or t-butyl group. Trialkylsilyl groups such as the tert-butyldimethylsilyl group or triisopropylsilyl group are particularly preferable since these trialkylsilyl groups are stable in preparing the β-lactam compound having the general formula (I) and can be removed in a considerably easy way after obtaining the desired β-lactam compound.

$R^2$, $R^3$ and $R^4$ in the general formula (I) are selected from an alkyl group of $C_1$ to $C_4$ such as methyl group, isopropyl group and tert-butyl group and $R^2$, $R^3$ and $R^4$ may be the same or different from each other. It is preferred that all of $R^2$, $R^3$ and $R^4$ are methyl groups, both $R^2$ and $R^3$ are methyl groups and $R^4$ is a t-butyl group or both $R^2$ and $R^3$ are phenyl groups and $R^4$ is a t-butyl group since, when such substitution groups are employed, the configuration at the 3-position in the β-lactam ring tends to become the preferable (R)-form.

With respect to the stereochemistry of the β-lactam compound having the general formula (I), three asymmetric carbon atoms are present and eight kinds of stereoisomers can be produced. However, it is preferred that the configuration is C-3(R), C-4(R), and (R) at the asymmetric carbon atom in the O-protected hydroxyethyl group.

The usefulness of the β-lactam compound having a silylether group at the 4-position is obvious from Reference Example 1, since said β-lactam can be converted into a useful intermediate, 3(R)-(1-hydroxyethyl)-4(R)-acetoxyazetidin-2-one, as shown in Reference Example 1.

The process of the present invention is illustrated in the following scheme:

Scheme I

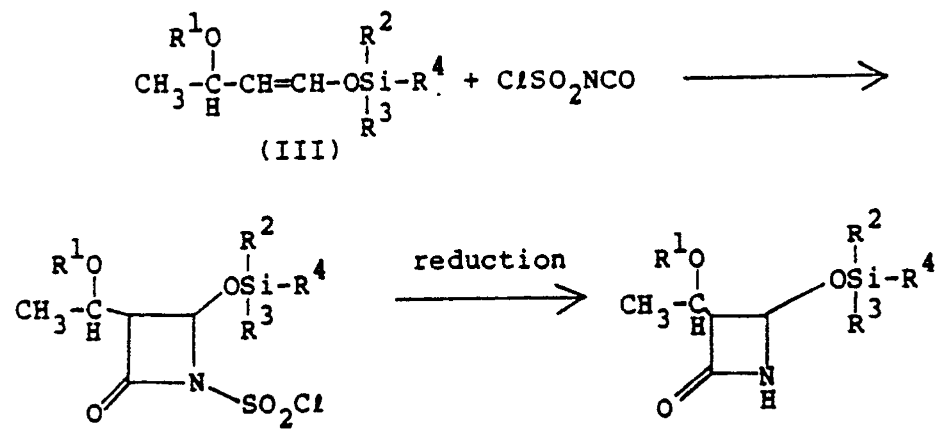


(III)

(I)

Wherein $R^1$, $R^2$, $R^3$ and $R^4$ are as above.

Examples of the enolsilylether employed as a starting material in the present invention are, for instance, 3-tert-butyldimethylsilyloxybuten-1-yl trimethylsilyl ether, 3-tert-butyldimethylsilyloxybuten-1-yl dimethylisobutylsilyl ether, 3-tert-butyldimethylsilyloxybuten-1-yl-tert-butyldimethylsilyl ether, 3-tert-butyldimethylsilyloxybuten-1-yl-tert-butylmethylphenylsilyl ether, 3-tert-butyldimethylsilyloxybuten-1-yl-tert-butyldiphenylsilyl ether, 3-triisopropylsilyloxybuten-1-yl trimethylsilylether, 3-triisopropylsilyloxybuten-1-yl-tert-butyldimethylsilyl ether, 3-isopropyldimethylsilyloxybuten-1-yl trimethylsilyl ether, 3-acetoxybuten-1-yl-trimethylsilyl ether, 3-acetoxybuten-1-yl-tert-butyldimethylsilyl ether, 3-acetoxybuten-1-yl-tert-butyldiphenylsilyl ether, 3-tert-butoxybuten-1-yl trimethylsilyl ether, 3-tert-butoxybuten-1-yl-tert-butyldimethylsilyl ether, 3-tert-butoxybuten-1-yl-tert-butyldiphenylsilyl ether, 3-benzyloxycarbonyloxybuten-1-yl trimethylsilyl ether, 3-benzyloxycarbonyloxybuten-1-yl-tert-butyldimethylsilyl ether, 3-benzyloxycarbonyloxybuten-1-yl-tert-butyldiphenyl ether, 3-p-nitrobenzyloxycarbonyloxybuten-1-yl trimethylsilyl ether, 3-p-nitrobenzyloxycarbonyloxybuten-1-yl-tert-butyldimethylsilyl ether, 3-p-nitrobenzyloxycarbonyloxybuten-1-yl-tert-butyldiphenylsilyl ether, 3-o-nitrobenzyloxycarbonyloxybuten-1-yl-trimethylsilyl ether, 3-o-nitrobenzyloxycarbonyloxybuten-1-yl-tert-butyldimethylsilyl ether, 3-o-nitrobenzyloxycarbonyloxybuten-1-yl-tert-butyldiphenylsilyl ether, preferably a enolsilylether having the general formula (III) wherein $R^1$ is the tert-butyldimethylsilyl group such as 3-tert-butyldimethylsilyloxybuten-1-yl trimethylsilyl ether, 3-tert-butyldimethylsilyoxybuten-1-yl-tert-butyldimethylsilyl ether, 3-tert-butyldimethylsilyloxybuten-1-yl-tert-butyldiphenylsilyl ether, 3-tert-butyldimethylsilyloxybuten-1-yl-tert-butylmethylphenylsilyl ether and 3-tert-butyldimethylsilyloxy-buten-1-yl dimethylisobutylsilyl ether. These materials can be prepared by the following scheme starting from 3-hydroxybutyric acid ester:

Scheme II

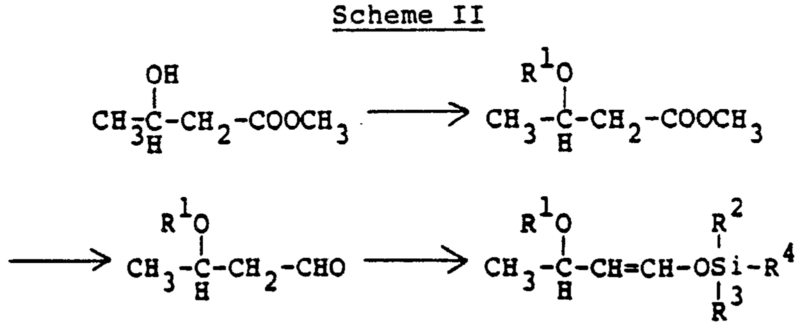

(III)

The configuration of O-protected hydroxyethyl group at the 3-position of the β-lactam compound (I) of the present invention is preferably (R). For the stereospecific configuration, the reaction of scheme II is conducted employing optically active enolsilylether which is obtained from optically active 3(R)-hydroxybutyric acid ester.

In the reaction of enolsilylether with chlorosulfonyl isocyanate to form the β-lactam ring, the configuration of the resulting β-lactam compound is dependent on the kind of the silylether group. Examples of the preferable silylether groups for obtaining the β-lactam compound (I) of the C-3(R), C-4(R) configuration which is suited for the synthesis of carbapenem β-lactam antibiotics such as thienamycin are, for instance, the trimethylsilyl group, tert-butyldiphenylsilyl group, tert-butyldimethylsilyl group, tert-butylmethylphenylsilyl group or dimethylisobutylsilyl group. $R^1$, which is a protective group for the hydroxy group of the enosilylether, is preferably the tert-butyldimethylsilyl group in a viewpoint of stereochemistry as mentioned above and a good reactivity with chlorosulfonylisocyanate. The reaction of the enolsilylether with chlorosulfonylisocyanate can be conducted either without any solvent or in the presence of an organic solvent which does not react with both chlorosulfonylisocyanate and enolsilylether, for instance, methylene chloride, toluene, tetrahydrofuran, n-hexane or ethylether. The reaction temperature is in the range from −70°C to around room temperature, preferably −40°C to 0°C and the reaction can be carried out by controlling the exothermic reaction and adding dropwise chlorosulfonylisocyanate to enolsilylether solution. The enolsilylether and chlorosulfonylisocyanate are used in such an amount that the molar ratio of these materials is around 1:1. The reaction time is in the range from ten minutes to several hours.

The obtained β-lactam compound having an N-sulfonyl chloride group is reduced and converted into the desired β-lactam compound. Examples of the reducing agent used in the above reduction are, for instance, metal hydrides such as lithium aluminum hydride, sodium boron hydride and sodium bis(2-methoxyethoxy)aluminum hydride, and Raney nickel. Thiol compounds such as thiophenol and alkylmercaptan can also be used as the reducing agent. The reduction is carried out in an organic solvent such as tetrahydrofuran, toluene or ethylether when the metal hydride such as lithium aluminum hydride is employed, and a base such as pyridine is used in the reaction together with the above organic solvent when a thiol compound is the reducing agent. The reaction temperature of the reduction is in the range from −40°C to 0°C. Lithium aluminum hydride or sodium bis(2-methoxyethoxy)aluminum hydride is preferably employed as the reducing agent.

After the reduction is completed, water is added to the reaction system. The desired β-lactam compound is extracted with an organic solvent such as ethylether, toluene or ethylacetate and dried with a dehydrating agent such as magnesium sulfate. The solvent is distilled away under reduced pressure to give the β-lactam compound having an O-protected hydroxyethyl group at the 3-position and a silylether group at the 4-position. If necessary, the thus obtained β-lactam compound can be isolated and purified by column chromatography.

The present invention is more particularly explained by the following Examples and Reference Examples. Hyflo Super-Cel® is a filter aid available from Johns-Manville Sales Corp., whose main constituent is diatomaceous earth.

## Example 1

[Preparation of (3R, 4R, 5R)-3-(1-tert-butyldimethylsilyloxyethyl)-4-trimethylsilyloxyazetidin-2-one]

One gram of (3R)-3-tert-butyldimethylsilyloxybute-1-nyl trimethylsilyl ether (a mixture of the trans-form and the cis-form in a ratio of 9:1) was added to 5 ml of ether, and thereto 0.3 ml of chlorosulfonylisocyanate was added with stirring and cooling to −50°C under nitrogen gas for 20 minutes. The reaction mixture was slowly warmed to −40°C and stirred further for 30 minutes. Then the reaction mixture was again cooled to −60°C and thereto 0.08 g of $LiAlH_4$ was added. The resulting mixture was slowly warmed to −40°C and stirred for 60 minutes. The resulting mixture was then quickly transferred to a mixed solution of 150 ml of ice-water and 100 ml of ether and stirred for 30 minutes. After completion of the stirring, the insoluble portion was removed by Hyflo Super-Cel. The ether layer was washed with a saturated solution of salt and dried with magnesium sulfate. Then ether was distilled away under reduced pressure to give 0.6 g of the liquid of desired β-lactam as a mixture of the (3R, 4R, 5R)-form and the (3S, 4S, 5R)-form (10:1). The obtained β-lactam was treated with silica-gel column-chromatography (hexane:methylene chloride = 50:1) and a portion which predominantly contained the (3R, 4R, 5R)-form was collected and concentrated to give 0.3 g of the desired product as a semisolid.

$[\alpha]_D^{25} = -12.4°C$ (C = 1, $CCl_4$).

$^1$HNMR (90 MHz, $CCl_4$) δ (ppm):

0.08 (6H, S), 0.18 (9H, S), 0.88 (9H, S), 1.25 (3H, d), 2.97 (1H, dd), 4.17 (1H, m), 5.37 (1H, d) and 6.60 (1H, broad).

## Example 2

[Preparation of (3R, 4R, 5R)-3-(1-tert-buthyldimethylsilyloxyethyl)-4-tert-butyldimethylsilyloxyazetidin-2-one]

One gram of (3R)-3-tert-butyldimethylsilyloxybute-1-nyl tert-butyldimethylsilyl ether (a mixture of the trans-form and the cis-form in a ratio of 3:2) was added to 8 ml of ether, the mixture was cooled to −50°C under nitrogen gas and thereto 0.25 ml of chlorosulfonylisocyanate was added with stirring and cooling to −50°C under nitrogen gas for 10 minutes. The reaction mixture was slowly warmed to −20°C and stirred for 20 minutes. Then the reaction mixture was again cooled to −60°C and thereto 0.066 g of $LiAlH_4$ was added. The resulting mixture was again slowly warmed to −40°C and stirred for 60 minutes. The resulting mixture was then quickly poured into a mixed solution of 100 ml of ice-water and 100 ml of ether and the mixture

was stirred for 30 minutes. After completion of the stirring, the separated ether layer was successively washed with a 5% aqueous solution of $NaHCO_3$, an aqueous solution of hydrochloric acid of pH 3 and a saturated solution of salt and dried with magnesium sulfate. The solvent was distilled away to give 0.9 g of the desired β-lactam as a mixture of the (3R, 5R)-form and the (3S, 5R)-form, whose ratio was 3:2.

The obtained mixture was purified with silica-gel column-chromatography (hexane:ethyl acetate = 10:1) to give 0.25 g of the desired (3R, 4R, 5R) β-lactam and 0.13 g of the (3S, 4S, 5R) β-lactam as a solid.

The β-lactams had the following properties:

(3R, 4R, 5R)-form
$[\alpha]_D^{25} = -8.3°$ (C = 1, $CCl_4$).
$^1$HNMR (90 MHz, $CDCl_4$) δ (ppm):
0.08 ($CH_3$ X 2, s), 0.13 ($CH_3$ X 2, s), 0.90 (9H, s), 0.93 (9H, s), 1.27 ($CH_3$, d), 2.95 (1H, dd), 4.13 (1H, m), 5.37 (1H, d) and 6.13 (NH, broad).
mp: 131° to 133°C;

(3S, 4S, 5R)-form
$[\alpha]_D^{25} = -33.1°$ (C = 1, $CCl_4$).
$^1$HNMR (90 MHz, $CDCl_4$) δ (ppm):
0.10 ($CH_3$ X 2, s), 0.13 ($CH_3$ X 2, s), 0.08 (9H, s), 0.90 (9H, s), 1.31 ($CH_3$, d), 3.05 (1H, dd), 4.22 (1H, m), 5.27 (1H, d) and 6.37 (1H, broad).
mp: 43° to 45°C.

Example 3

[Preparation of (3R, 4R, 5R)-3-(1-tert-butyldimethylsilyloxyethyl)-4-tert-butyldimethylsilyloxyazetidin-2-one]

One gram of (3R)-3-tert butyldimethylsilyloxybute-1-nyl tert-butyldimethylsilyl ether (a mixture of the trans-form and the cis-form in a ratio of 3:2) was added to 8 ml of hexane, and thereto 0.25 ml of chlorosulfonylisocyanate was added with stirring and cooling to −50°C under nitrogen gas for 10 minutes. The reaction mixture was slowly warmed to −20°C and stirred for 20 minutes. Then the reaction mixture was again cooled to −60°C and thereto a solution of 0.7 g of thiophenol in 2 ml of hexane was added. After the solution was stirred for 10 minutes, a solution of 0.4 g of pyridine in 2 ml of hexane was further added thereto. The mixture was slowly warmed to room temperature with stirring, and thereto 50 ml of hexane was added. Then the resulting mixture was successively washed with a 5% aqueous solution of $NaHCO_3$, an aqueous solution of hydrochloric acid of pH 3 and a saturated solution of salt and dried with magnesium sulfate. The hexane was distilled away under reduced pressure to give 0.7 g of the desired β-lactam as a mixture of the (3R, 4R, 5R)-form and the (3S, 4S, 5R)-form, whose ratio was 3:2.

The obtained mixture was separated and purified by silica-gel column-chromatography (hexane:ethyl acetate = 10:1) to give 0.18 g of the desired (3R, 4R, 5R) β-lactam and 0.10 g of the (3S, 4S, 5R) β-lactam.

The properties of each β-lactam agreed with those shown in Example 2.

Example 4

[Preparation of (3R, 4R, 5R)-3-(1-tert-butyldimethylsilyloxyethyl)-4-tert-butylmethylphenylsilyloxyazetidin-2-one]

One gram of (3R)-3-tert butyldimethylsilyloxybute-1-nyl tert-butylmethylphenylsilyl ether (a mixture of the trans-form and the cis-form in a ratio of 7:1) was added to 5 ml of ether, the mixture was cooled to −50°C under nitrogen gas and thereto 0.22 ml of chlorosulfonylisocyanate was added with stirring and cooling to −50°C under nitrogen gas for 10 minutes. The reaction mixture was slowly warmed to −40°C and stirred further for 30 minutes. Then the reaction mixture was again cooled to −60°C and thereto 0.06 g of $LiAlH_4$ was added. The mixture was slowly warmed to −45°C and stirred for 40 minutes. The mixture was then quickly transferred to a mixture of 150 ml of ice-water and 100 ml of ether and stirred for 30 minutes. After completion of the stirring, the obtained mixture was separated and the organic layer was successively washed with a 5% aqueous solution of $NaHCO_3$, an aqueous solution of hydrochloric acid and a saturated solution of salt and dried with magnesium sulfate. The solvent was distilled away to give 0.98 g of a crude product as an oil.

The obtained β-lactam had the following properties.

(3R, 4R, 5R)-form
NMR ($CDCl_3$) δ (ppm):
0.00 (6H, s), 0.43 (3H, s), 0.80 (9H, s), 0.90 (9H, s), 1.19 (3H, d), 2.99 (1H, dd), 4.10 (1H, m), 5.35 (1H, d), 6.63 (1H, d) and 7.37 (5H, m).

Example 5

[Preparation of (3R, 4R, 5R)-3-(1-tert-butyldimethylsilyloxy)-4-dimethylisobutylsilyloxyazetidin-2-one]

One gram of (3R)-3-tert-butyldimethylsilyloxybute-1-nyl dimethylisobutylsilyl ether (a mixture of the

trans-form and the cis-form in a ratio of 5:1) was added to 6 ml of ether, and thereto 0.26 ml of chlorosulfonylisocyanate was added with stirring and cooling to −60°C under nitrogen gas for 10 minutes. The reaction mixture was slowly warmed to −50°C and stirred further for 30 minutes. Then the reaction mixture was again cooled to −60°C and thereto 0.066 g of $LiAlH_4$ was added. The mixture was slowly warmed to −50°C and stirred for 60 minutes. The reaction mixture was quickly transferred to a mixture of 150 ml of ice-water and 100 ml of ether and the mixture was stirred for 30 minutes. After completion of the stirring, the obtained mixture was separated, the organic layer was successively washed with a 5% aqueous solution of $NaHCO_3$, an aqueous solution of hydrochloric acid and a saturated solution of salt and dried with magnesium sulfate. The solvent was distilled away to give 0.59 g of a crude product as an oil.

The obtained β-lactam had the following properties.

(3R, 4R, 5R)-form

NMR ($CDCl_3$) δ (ppm):

0.03 (6H, s), 0.15 (6H, s), 0.60 (2H, d), 0.87 (9H, s), 0.93 (6H, d), 1.22 (3H, d), 1.80 (1H, m), 2.85 (1H, dd), 4.15 (1H, m), 5.26 (1H, d) and 7.77 (1H, broad s).

Example 6

[Preparation of (3R, 4R, 5R)-3-(1-tert-butyldimethylsilyloxy)-4-trimethylsilyloxyazetidin-2-one]

One gram of (3R)-3-tert-butyldimethylsilyloxybute-1-nyl trimethylsilyl ether (a mixture of the trans-form and the cis-form in a ratio of 9:1) was added to 5 ml of ether, and thereto 0.3 ml of chlorosulfonylisocyanate was added dropwise with stirring and cooling to −50°C under nitrogen gas for 20 minutes. After the mixture was stirred for 90 minutes at −50°C, it was cooled to −70°C and thereto a solution where 178 mg of aluminum chloride and 152 mg of sodium boron hydride were dissolved in 8 ml of diglyme was added. The mixture was slowly warmed to −60°C and stirred for 1 hour, which was quickly transferred to an ice-cooled mixture of a 0.5 M aqueous solution of Rochelle salt and 100 ml of hexane and the mixture was stirred for 20 minutes under cooling with ice. After completion of the stirring, the insoluble portion was filtered with Hyflo Super-Cel® and the hexane layer was dried with magnesium sulfate. The solvent was distilled away to give 0.3 g of the desired β-lactam which predominantly contained the (3R, 4R, 5R)-form.

Example 7

[Preparation of (3R, 4R, 5R)-3-(1-tert-butyldimethylsilyloxyethyl)-4-trimethylsilyloxyazetidin-2-one]

One gram of (3R)-3-tert-butyldimethylsilyloxybute-1-nyl trimethylsilyl ether (a mixture of the trans-form and the cis-form in a ratio of 9:1) was added to 5 ml of toluene, and thereto 0.32 ml of chlorosulfonylisocyanate was added dropwise with stirring and cooling to −50°C under nitrogen gas for 10 minutes. After the mixture was stirred for 2 hours at −50°C, it was cooled to −70°C, and thereto 11 ml of toluene and then 4 ml of a toluene solution containing 1M sodium bis(2-methoxyethoxy)aluminum hydride were added. The mixture was slowly warmed to −50°C and stirred for 60 minutes. The reaction mixture was quickly transferred to an ice-cooled mixture of 100 ml of 0.5 M aqueous solution of Rochelle salt and 100 ml of toluene and the mixture was stirred for 30 minutes under cooling with ice. After completion of the stirring, the insoluble portion was filtered with Hyflo Super-Cel®, and the toluene layer was washed with a saturated solution of salt and dried with magnesium sulfate. The toluene was distilled away under reduced pressure to give 0.81 g of white crystal of the desired β-lactam containing the (3R, 4R, 5R)-form and the (3S, 4S, 5R)-form in a ratio of 10:1.

Example 8

[Preparation of (3R, 4R, 5R)-3-(1-isopropyldimethylsilyloxyethyl)-4-trimethylsilyloxyazetidin-2-one]

One gram of (3R)-3-isopropyldimethylsilyloxybute-1-nyl trimethylsilyl ether (a mixture of the trans-form and the cis-form in a ratio of 8:1) was added to 5 ml of ether, and thereto 0.3 ml of chlorosulfonylisocyanate was added with stirring and cooling to −60°C under nitrogen gas for 25 minutes. After the mixture was stirred for 2 hours at −60°C, it was cooled to −70°C, and thereto 10 ml of toluene and then 4 ml of a toluene solution containing 1M sodium bis(2-methoxyethoxy)aluminum hydride were added. The mixture was slowly warmed to −50°C for 60 minutes and stirred for 60 minutes. The reaction mixture was quickly transferred to an ice cooled mixture of 50 ml of a 0.5 M aqueous solution of Rochell salt and 50 ml of toluene and the resulting mixture was stirred for 30 minutes under cooling with ice. After completion of the stirring, the insoluble portion was filtered with Hyflo Super-Cel® and the toluene layer was washed with a saturated solution of salt and dried with magnesium sulfate. The toluene was distilled away under reduced pressure to give 0.61 g of the desired β-lactam which predominantly contained the (3R, 4R, 5R)-form as an oil.

$^1$HNMR (90 MHz, $CCl_4$) δ (ppm):

0.07 (6H, s), 0.92 (7H), 1.21 (3H, d), 2.85 (1H, dd), 4.05 (1H, m), 5.21 (1H, d) and 7.31 (NH).

Reference Example 1

[Preparation of (3R, 4R)-4-acetoxy-3-[(R)-1-tert-butyldimethylsilyloxyethyl]-azetidin-2-one]

One gram of (3R, 4R, 5R)-3-(1-tert-butyldimethylsilyloxyethyl)-4-trimethylsilyloxyazetidin-2-one was

dissolved in 10 ml of DMF, thereto 0.89 g of triethylamine and 0.61 g of tert-butyldimethylsilyl chloride were added and the mixture was stirred for 9 hours at room temperature. After completion of the reaction, DMF was distilled away under reduced pressure and thereto 30 ml of hexane was added. The solution was successively washed with a 2.5% aqueous solution of $NaHCO_3$, an aqueous solution of hydrochloric acid of pH 3 and a saturated solution of salt and dried with magnesium sulfate. The solvent was distilled away to give 1.24 g of the liquid of the crude product. There was added 1.0 g of the obtained liquid to 5 ml of methylene chloride, and thereto 0.85 g of dimethylaminopyridine and 1.1 ml of acetic anhydride were further added and the mixture was reacted for 6 hours at room temperature. After the solution was washed successively with a 5% aqueous solution of $NaHCO_3$, an aqueous solution of hydrochloric acid of pH 3 and a saturated solution of salt and dried with magnesium sulfate, the solvent was distilled away to give 0.8 g of the liquid of the crude product, which was purified by silica-gel column-chromatography (benzene:hexane = 2:1) to give 0.5 g of (3R, 4R, 5R)-4-acetoxy-1-(tert-butyldimethylsilyl)-3-(1-tert-butyldimethylsilyloxyethyl)-azetidin-2-one as a liquid. There was added 0.5 g of the obtained liquid to 2 ml of THF and, to which 2 ml of THF dissolving 0.4 g of tetrabutylammonium fluoride and 0.17 g of acetic acid were added. The mixture was stirred for 30 minutes at room temperature, thereto 20 ml of ethyl acetate was added and the mixture was successively washed with a 5% aqueous solution of $NaHCO_3$ and a saturated solution of salt, dried with magnesium sulfate and the solvent was distilled away to give 0.30 g of crystals of the desired product. The obtained crystals were purified by silica-gel column-chromatography (benzene:ethyl acetate = 6:1) to give 0.27 g of the desired β-lactam as a solid.

The obtained β-lactam had the following properties.

mp: 107° to 108°C.

$[\alpha]_D^{25}$ = +50° (C = 0.5, $CHCl_3$).

$^1$HNMR (90 $MH_2$, $CDCl_3$) δ (ppm):

0.08 (6H, s), 0.84 (9H, s), 1.20 (3H, d), 2.01 (3H, s), 3.04 (1H, dd), 4.12 (1H, m), 5.76 (1H, d) and 6.73 (NH).

Reference Example 2

[Preparation of (3R)-3-tert-butyldimethylsilyloxybuten-1-yl trimethylsilyl ether]

There was added 8.0 g of (3R)-3-tert-butyl-dimethylsilyloxybutylaldehyde to 40 ml of methylene chloride, 8.0 g of triethylamine and 12 ml of trimethylsilyl chloride were added to the above solution and the mixture was refluxed for 13 hours. After completion of the reaction, the solvent was distilled away and thereto n-hexane was added. The resultant was successively washed with a 5% aqueous solution of $NaHCO_3$, an aqueous solution of hydrochloric acid of pH 3 and a saturated solution of salt, dried with magnesium sulfate and the solvent was distilled away to give 10 g of the liquid. The obtained liquid was distilled away under reduced pressure to give 8.0 g of the desired product (bp 85°C/3 mmHg). The obtained product was a mixture of the trans-form and the cis-form in a ratio of 15:1.

$[\alpha]_D^{25}$ = −3.2° (C = 1, $CCl_4$).

$^1$HNMR (90 $MH_2$, $CCl_4$) (Trans-form) δ (ppm):

0.11 (6H, s), 0.25 (9H, s), 0.90 (9H, s), 1.25 (3H, d), 4.25 (1H, m), 4.95 (1H, dd) and 6.28 (1H, d).

Reference Example 3

[Preparation of (3R)-3-tert-butyldimethylsilyloxybuten-1-yl-tert-butyldimethylsilyl ether]

There was added 8.0 g of (3R)-3-tert-butyldimethylsilyloxybutylaldehyde to 50 ml of DMF, to which 12.0 g of tert-butyldimethylsilyl chloride and 16.0 g of triethylamine were added and the mixture was stirred for 13 hours at 90°C. After completion of the reaction, DMF was distilled away under reduced pressure and thereto 100 ml of n-hexane was added, which was successively washed with a 5% aqueous solution of $NaHCO_3$, an aqueous solution of hydrochloric acid of pH 3 and a saturated solution of salt, dried with magnesium sulfate and the solvent was distilled away to give 8.0 g of a liquid. The obtained liquid was distilled away under reduced pressure to give 5.0 g of the desired product (bp 65°C/1 mmHg). The obtained product was a mixture of the trans-form and the cis-form in a ratio of 3:2.

$[\alpha]_D^{25}$ = −14.96° (C = 2, $CCl_4$).

$^1$HNMR (90 MHz, $CCl_4$) δ (ppm):

Trans-form

0.07 (6H, s), 0.17 (6H, s), 0.90 (9H, s), 0.97 (9H, s), 1.20 (3H, d), 4.20 (1H, m), 4.95 (1H, dd) and 6.26 (1H, d).

Cis-form

0.07 (6H, s), 0.17 (6H, s), 0.90 (9H, s), 0.97 (9H, s), 1.15 (3H, d), 4.20 (1H, m), 4.48 (1H, dd), and 5.97 (1H, d).

Reference Example 4

[Preparation of (3R)-3-tert-butyldimethylsilyloxybuten-1-yl-tert-butylmethylphenylsilyl ether]

There was added 10.0 g of (3R)-3-tert-butyldimethylsilyloxybutylaldehyde to 40 ml of DMF. To this solution 6.6 g of dimethylaminopyridine and 10.4 g of tert-butylmethylphenylsilyl chloride were added and the mixture was stirred for 14 hours at 75°C. After completion of the reaction, DMF was distilled away under reduced pressure and thereto 100 ml of n-hexane was added, which was successively washed with a 5%

aqueous solution of $NaHCO_3$, an aqueous solution of hydrochloric acid of pH 3 and a saturated solution of salt, dried with magnesium sulfate and the solvent was distilled away to give 10.2 g of a liquid. The obtained liquid was purified by silica-gel column-chromatography (hexane:ethyl acetate = 20:1) to give 7.2 g of the desired product. The obtained product was a mixture of the trans-form and the cis-form in a ratio of 7:1.

$[\alpha]_D^{25} = -5.3°$ (C = 1, $CCl_4$).

$^1$HNMR (90 MHz, $CDCl_3$) δ (ppm):

Trans-form

0.00 (6H, s), 0.40 (3H, s), 0.85 (9H, s), 0.93 (9H, s), 1.18 (3H, d), 4.20 (1H, m), 5.06 (1H, dd), 6.35 (1H, d) and 7.40 (5H, m).

Reference Example 5

[Preparation of (3R)-3-tert-butyldimethylsilyloxybuten-1-yl dimethylisobutylsilyl ether]

There was added 20.0 g of (3R)-3-tert-butyldimethylsilyloxybutylaldehyde to 80.0 ml of DMF, 16.3 ml of TMEDA and 14.8 g of isobutyldimethylsilyl chloride were added to the above solution and the mixture was stirred for 3 hours at 80°C. After completion of the reaction, the solvent was distilled away and thereto n-hexane was added, which was successively washed with a 5% aqueous solution of $NaHCO_3$, an aqueous solution of hydrochloric acid of pH 3 and a saturated solution of salt, dried with magnesium sulfate and the solvent was distilled away to give 16.5 g of the liquid. The obtained liquid was purified by silica-gel column-chromatography (n-hexane:ethyl acetate = 20:1) to give 10.6 g of the desired product. The obtained product was a mixture of the trans-form and the cis-form in a ratio of 5:1.

$[\alpha]_D^{25} = -6.0°$ (C = 1, $CCl_4$).

$^1$HNMR (90 MNz, $CDCl_3$) δ (ppm):

Trans-form

0.03 (6H, s), 0.15 (6H, s), 0.60 (2H, d), 0.85 (9H, s), 0.90 (6H, d), 1.15 (3H, d), 1.80 (1H, m), 4.20 (1H, m), 4.95 (1H, dd) and 6.25 (1H, d).

**Claims**

1. A β-lactam compound having the general formula (I):

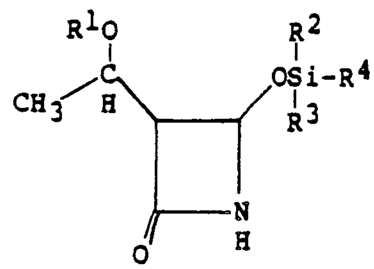

(I)

wherein $R^1$ is a protective group for hydroxy group, $R^2$, $R^3$ and $R^4$ are selected from an alkyl group of $C_1$ to $C_4$, a phenyl group and an aralkyl group.

2. The β-lactam compound of Claim 1, wherein $R^1$ is the general formula:

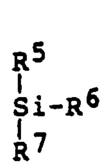

(II)

wherein $R^5$, $R^6$ and $R^7$ are an alkyl group of $C_1$ to $C_4$.

3. The β-lactam compound of Claim 1, wherein $R^1$ is the t-butyldimethylsilyl group.

4. A process for preparing a β-lactam compound having the general formula:

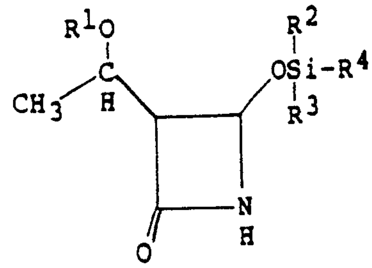

(I)

wherein $R^1$ is a protective group for hydroxy group, $R^2$, $R^3$ and $R^4$ are selected from an alkyl group of $C_1$ to $C_4$, a phenyl group and an aralkyl group, which comprises reacting an enolsilylether having the general formula:

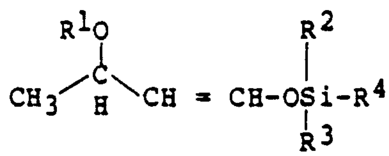

(III)

wherein $R^1$, $R^2$, $R^3$ and $R^4$ are as above, with chlorosulfonylisocyanate and then reducing the obtained product.

5. The process of Claim 4, wherein $R^1$ is the general formula:

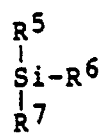

(II)

wherein $R^5$, $R^6$ and $R^7$ are an alkyl group of $C_1$ to $C_4$.

6. The process of Claim 4, wherein $R^1$ is the t-butyldimethylsilyl group.

7. The process of Claim 4, wherein the reducing agent employed is a metal hydride.

8. The process of Claim 7, wherein the reducing agent is lithium aluminum hydride.

9. The process of Claim 7, wherein the reducing agent is sodium bis(2-methoxyethoxy)aluminum hydride.

10. An enolsilylether having the general formula (III):
(I):

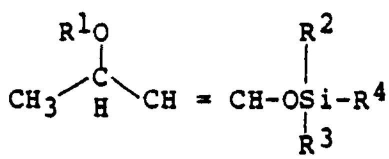

(III)

wherein $R^1$ is a protective group for hydroxy group, $R^2$, $R^3$ and $R^4$ are selected from an alkyl group of $C_1$ to $C_4$, a phenyl group and an aralkyl group.

11. The enolsilylether of Claim 10, wherein $R^1$ is the general formula (II):

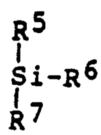

(II)

wherein $R^5$, $R^6$ and $R^7$ are selected from an alkyl group of $C_1$ to $C_4$.

12. The enolsilylether of Claim 10, wherein $R^1$ is the t-butyldimethylsilyl group.

**Patentansprüche**

1. β-Lactamverbindung der allgemeinen Formel (I):

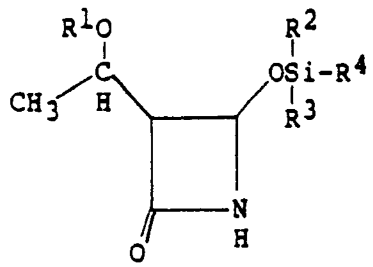

(I)

worin $R^1$ eine Schutzgruppe für Hydroxygruppe ist, $R^2$, $R^3$ und $R^4$ ausgewählt sind aus einer Alkylgruppe von $C_1$ bis $C_4$, einer Phenylgruppe und einer Aralkylgruppe.

2. β-Lactamverbindung nach Anspruch 1, worin $R^1$ die allgemeine Formel hat:

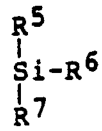

(II)

worin $R^5$, $R^6$ und $R^7$ eine Alkylgruppe von $C_1$ bis $C_4$ sind.

3. β-Lactamverbindung nach Anspruch 1, worin $R^1$ die t-Butyldimethylsilylgruppe ist.

4. Verfahren zur Herstellung einer β-Lactamverbindung mit der allgemeinen Formel:

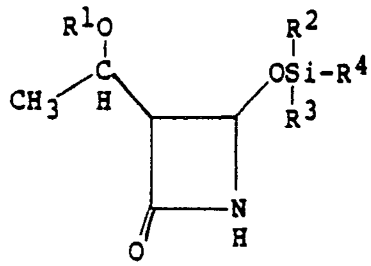

(I)

worin $R^1$ eine Schutzgruppe für Hydroxygruppe ist, $R^2$, $R^3$ und $R^4$ ausgewählt sind aus einer Alkylgruppe von $C_1$ bis $C_4$, einer Phenylgruppe und einer Aralkylgruppe, welches umfaßt Umsetzen eines Enolsilylethers mit der allgemeinen Formel:

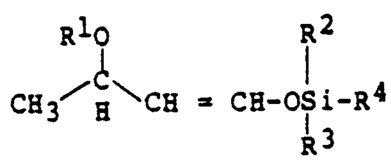

(III)

worin $R^1$, $R^2$, $R^3$ und $R^4$ wie oben sind, mit Chlorsulfonylisocyanat und dann Reduzieren des erhaltenen Produktes.

5. Verfahren nach Anspruch 4, worin $R^1$ die allgemeine Formel hat:

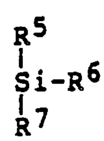

(II)

worin $R^5$, $R^6$ und $R^7$ eine Alkylgruppe von $C_1$ bis $C_4$ sind.

6. Verfahren nach Anspruch 4, worin $R^1$ die t-Butyldimethylsilylgruppe ist.

7. Verfahren nach Anspruch 4, worin das Reduktionsmittel, das verwendet wird, ein Metallhydrid ist.

8. Verfahren nach Anspruch 7, worin das Reduktionsmittel Lithiumaluminiumhydrid ist.

9. Verfahren nach Anspruch 7, worin das Reduktionsmittel Natrium-bis(2-Methoxyethoxy)aluminiumhydrid ist.

10. Enolsilylether mit der allgemeinen Formel (III):

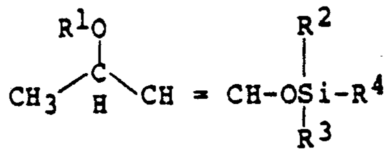

(III)

worin $R^1$ eine Schutzgruppe für Hydroxygruppe ist, $R^2$, $R^3$ und $R^4$ ausgewählt sind aus einer Alkylgruppe von $C_1$ von $C_4$, einer Phenylgruppe und einer Aralkylgruppe.

11. Enolsilylether nach Anspruch 10, worin $R^1$ die allgemeine Formel (II) hat:

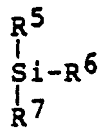

(II)

worin $R^5$, $R^5$ und $R^7$ ausgewählt sind aus einer Alkylgruppe von $C_1$ bis $C_4$.

12. Enolsilylether nach Anspruch 10, worin $R^1$ die t-Butyldimethylsilylgruppe ist.

**Revendications**

1. Composé β-lactame répondant à la formule générale (I):

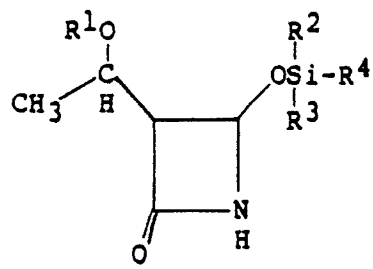

(I)

dans laquelle $R^1$ est un groupe protecteur de groupe hydroxy, $R^2$, $R^3$ et $R^4$ sont choisis parmi un groupe alkyle en $C_1$ à $C_4$, un groupe phényle et un groupe arylalkyle.

2. Composé β-lactame selon la revendication 1, dans lequel $R^1$ a pour formule générale:

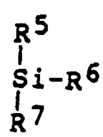

(II)

dans laquelle $R^5$, $R^6$ et $R^7$ sont un gorupe alkyle en $C_1$ à $C_4$.

3. Composé β-lactame selon la revendication 1, dans lequel $R^1$ est le groupe t-butyldiméthylsilyle.

4. Procédé de préparation d'un composé β-lactame répondant à la formule générale:

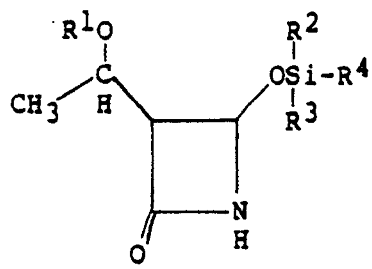

(I)

dans laquelle $R^1$ est un groupe protecteur de groupe hydroxy, $R^2$, $R^3$ et $R^4$ sont choisis parmi un groupe alkyle en $C_1$ à $C_4$, un groupe phényle et un groupe arylalkyle, qui comprend la réaction d'un éther d'énolsilyle répondant à la formule générale:

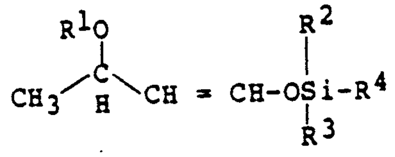

(III)

dans laquelle $R^1$, $R^2$, $R^3$ et $R^4$ sont tels que ci-dessus, avec de l'isocyanate de chlorosulfonyle, puis la réduction du produit obtenu.

5. Procédé selon la revendication 4, dans lequel $R^1$ a pour formule générale:

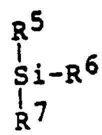

(II)

dans laquelle $R^5$, $R^6$ et $R^7$ sont un groupe alkyle en $C_1$ à $C_4$.

6. Procédé selon la revendication 4, dans lequel $R^1$ est le groupe t-butyldiméthylsilyle.

7. Procédé selon la revendication 4, dans lequel l'agent réducteur employé est un hydrure métallique.

8. Procédé selon la revendication 7, dans lequel l'agent réducteur est l'aluminohydrure de lithium.

9. Procédé selon la revendication 7, dans lequel l'agent réducteur est le bis(2-méthoxyéthoxy)aluminohydrure de sodium.

10. Ether d'énolsilyle répondant à la formule générale (III):

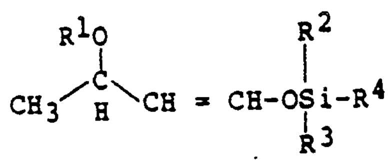

(III)

dans laquelle $R^1$ est un groupe protecteur de groupe hydroxy, $R^2$, $R^3$ et $R^4$ sont choisis parmi un groupe alkyle en $C_1$ à $C_4$, un groupe phényle et un groupe arylalkyle.

11. Ether d'énolsilyle selon la revendication 10, dans lequel $R^1$ répond à la formule générale (II):

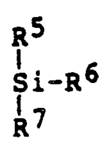

(II)

dans laquelle $R^5$, $R^6$ et $R^7$ sont choisis parmi les groupes alkyle en $C_1$ à $C_4$.

12. Ether d'énolsilyle selon la revendication 10, dans lequel $R^1$ est le groupe t-butyldiméthylsilyle.